(19) Europäisches Patentamt
European Patent Office
Office européen des brevets

(11) Publication number: **0 004 414**
**B1**

(12) # EUROPEAN PATENT SPECIFICATION

(45) Date of publication of patent specification: **24.07.85**

(21) Application number: **79300251.0**

(22) Date of filing: **20.02.79**

(51) Int. Cl.$^4$: **A 01 N 43/40,** A 01 N 43/64, A 01 N 43/48, C 07 D 213/64

(54) **Mixtures of herbicidal pyridyloxyphenoxypropanoic acid derivatives with other herbicides, and processes of killing unwanted plants therewith.**

(30) Priority: **01.03.78 GB 812878**

(43) Date of publication of application:
**03.10.79 Bulletin 79/20**

(45) Publication of the grant of the patent:
**24.07.85 Bulletin 85/30**

(84) Designated Contracting States:
**BE CH DE FR GB IT LU NL SE**

(56) References cited:
**EP-A-0 000 483**
**EP-A-0 001 473**
**BE-A- 862 325**
**FR-A-2 277 811**
**FR-A-2 288 089**
**FR-A-2 398 059**

**The file contains technical information submitted after the application was filed and not included in this specification**

(73) Proprietor: **IMPERIAL CHEMICAL INDUSTRIES PLC**
**Imperial Chemical House Millbank**
**London SW1P 3JF (GB)**

(72) Inventor: **Cartwright, David**
**1 Stonehaven Drive**
**Woodley Reading Berkshire (GB)**

(74) Representative: **Beton, John Lonsdale et al**
**Imperial Chemical Industries PLC**
**Legal Department: Patents PO Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 004 414 B1**

**Description**

This invention relates to herbicidal compositions which are a mixture of pyridyloxy phenoxypropionic acids with other herbicides, and a process for inhibiting plant growth using them.

In our European Patent Application No. 78300203.3 (Publication No. 1473) we disclose *inter alia* herbicidal pyridine compounds of the formula (I):

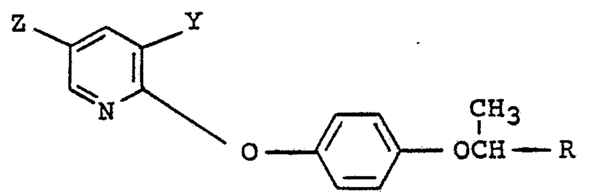

(I)

wherein at least one of Y and Z is trifluoromethyl, the other being hydrogen, fluorine, chlorine, bromine, iodine or trifluoromethyl, and R is a carboxyl group or a derivative thereof, such as a salt, ester, carboxamide or nitrile.

Examples of such compounds include those in which Z is a $CF_3$ radical, Y is a hydrogen or chlorine atom and R is carboxyl in the form of an ester thereof, in particular a $C_1$—$C_6$ alkyl ester.

Particular examples of such compounds include those listed in Table (I) below:

TABLE I

| COMPOUND NO | Z | Y | R |
|---|---|---|---|
| 1 | $CF_3$ | H | $CO.OC_2H_5$ |
| 2 | $CF_3$ | H | CO.OH |
| 3 | $CF_3$ | H | $CO.OC_5H_{11}$ |
| 4 | $CF_3$ | Cl | $CO.OC_3H_7$ |
| 5 | $CF_3$ | Cl | $CO.OC_2H_5$ |
| 6 | $CF_3$ | H | $CO.OC_4H_9$ |

These compounds are herbicides which are in general substantially more effective against grass species than against broad-leaved species of plant. They may be used to control unwanted grass species growing alone, or at suitable rates of application they may be used to control grass weeds growing among broad-leaved crop plants. The compounds may be either applied to the soil before the emergence of the unwanted grass species (pre-emergence application) or to the above-ground parts of growing grass plants (post-emergence application).

Compounds of this type are also disclosed in European Patent Application No. 78100291.0 (Publication No. 483).

In several agronomic situations the power of these herbicides to control grass weeds may not be sufficient to protect a crop. Accordingly, this invention provides a herbicidal composition characterised by comprising a compound of the formula (I) above, wherein at least one of Y and Z is trifluoromethyl, the other being hydrogen, fluorine, chlorine, bromine, iodine or trifluoromethyl and R is a carboxyl group or a salt or ester thereof or a carboxamido or cyano group, in admixture with at least one other herbicide as identified hereinbelow, the composition containing 0.1 to 10, preferably 0.5 to 2, parts by weight of the herbicide of formula (I) per part of said other herbicide.

The other herbicide is a herbicide having a complementary action to the compound of the formula (I) and can be a herbicide active against broad-leaved weeds or it can be a contact herbicide.

Specifically the other herbicide is chosen from:

A. Benzo-2,1,3-thiadiazin-4-one-2,2-dioxides, e.g. those of formula:

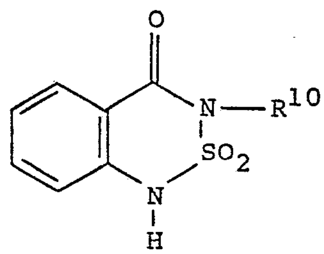

where $R^{10}$ is $C_1$—$C_6$ alkyl, in particular the compound in which $R^{10}$ is isopropyl, common name bentazon.

B. Phenoxyalkanoic acids and their derivatives (salts, esters, amides and the like).
Examples of such acids are:
4-chloro-2-methylphenoxyacetic acid (common name MCPA)
2-(2,4-dichlorophenoxy) propionic acid (common name dichlorprop)
2,4,5-trichlorophenoxyacetic acid (common name 2,4,5-T)
4-(4-chloro-2-methylphenoxy)butyric acid (common name MCPB)
2,4-dichlorophenoxyacetic acid (common name 2,4D)
4-(2,4-dichlorophenoxy)butyric acid (common name 2,4DB)
2-(4-chloro-2-methylphenoxy)propionic acid (common name mecoprop)

C. 3-[4-(4-halophenoxy)phenyl]-1,1 dialkyl ureas, in particular the compound 3-[4-(4-chlorophenoxy)-phenyl]-1,1-dimethylurea (common name chloroxuron).

D. Dinitrophenols, for example those of formula:

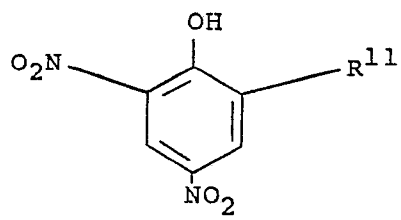

where $R^{11}$ is an alkyl group of 1 to 5 carbon atoms, and their derivatives, e.g. acetates; in particular such compounds wherein $R^{11}$ is methyl (common name DNOC), *tert*-butyl (common name dinoterb); or wherein $R^{11}$ is *sec*-butyl (common name dinoseb); and its ester dinoseb acetate.

E. Dinitroaniline herbicides, for example those of formula:

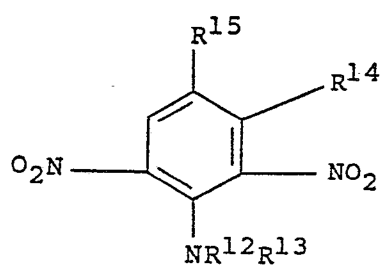

wherein $R^{12}$ is hydrogen or $C_1$—$C_6$ alkyl, cycloalkyl or alkenyl, optionally substituted with halogen; $R^{13}$ is $C_1$—$C_6$ alkyl; $R^{14}$ is hydrogen, methyl or amino; and $R^{15}$ is trifluoromethyl, $C_1$—$C_6$ alkyl, methyl sulphonyl or aminosulphonyl; in particular:
N'N'-diethyl-2,6-dinitro-4-trifluoromethyl-*m*-phenylene diamine (common name dinitramine) 2,6-dinitro-NN-dipropyl-4-trifluoromethylaniline (common name trifluralin)
4-methylsulphonyl-2,6-dinitro-NN-dipropylaniline (common name nitralin).

F. Phenylurea herbicides, e.g. of formula:

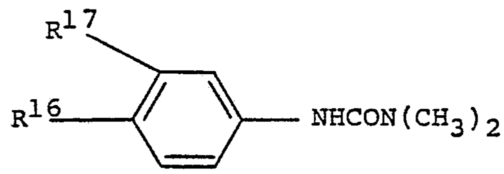

where $R^{16}$ and $R^{17}$ are independently hydrogen, chloro, $C_1$—$C_4$ alkyl or alkoxy, or trifluoromethyl; in particular the compound in which $R^{16}$ and $R^{17}$ are both chloro (common name diuron), the compound in which $R^{16}$ is hydrogen and $R^{17}$ is trifluoromethyl (common name fluometuron).

G. Phenylcarbamoyloxyphenylcarbamates, e.g. those of formula:

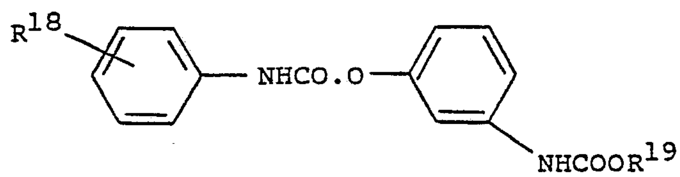

where $R^{18}$ is hydrogen or $C_{1-4}$ alkyl and $R^{19}$ is $C_1$—$C_4$ alkyl; in particular the compound where $R^{18}$ is *m*-methyl and $R^{19}$ is methyl (common name phenmedipham), and the compound where $R^{18}$ is hydrogen and $R^{19}$ is ethyl (common name desmedipham).

H. 2-phenylpyridazin-3-ones, e.g. those of formula:

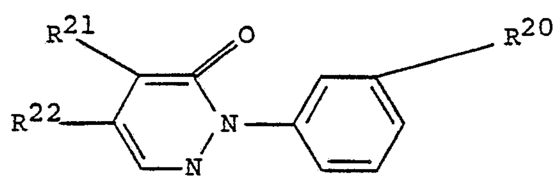

where $R^{20}$ is hydrogen or trifluoromethyl, $R^{21}$ is chloro, bromo or methoxy and $R^{22}$ is amino (optionally methyl substituted) or methoxy; particularly 5-amino-4-chloro-2-phenylpyridazine-3-one (common name pyrazon).

I. Uracil herbicides, for example of formula:

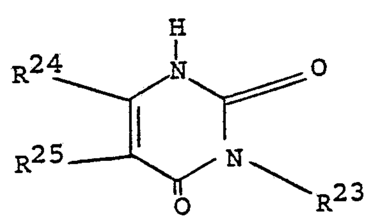

wherein $R^{23}$ is $C_3$—$C_5$ alkyl (preferably branched) or cyclohexyl, $R^{24}$ is methyl, $R^{25}$ is chlorine or bromine; or $R^{24}$ and $R^{25}$ together represent a $C_3$—$C_4$ alkylene bridge; particularly:
3-cyclohexyl-5,6-trimethylene uracil (common name lenacil)
5-bromo-3-*sec*-butyl-6-methyluracil (common name bromacil)
3-*tert*-butyl-5-chloro-6-methyluracil (common name terbacil)

J. 1,3,5-Triazine herbicides, for example of formula:

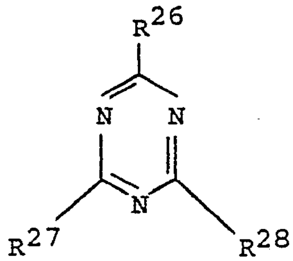

wherein $R^{26}$ is chloro, methoxy, methylthio or ethylthio; $R^{27}$ is ($C_1$—$C_6$) alkylamino, alkoxyalkylamino or dialkylamino; and $R^{28}$ is ($C_1$—$C_6$) alkylamino, alkoxyalkylamino, cyanoalkylamino, dialkylamino or azido; particularly:
2-chloro-4-ethylamino-6-*iso*propylamino-1,3,5-triazine (common name atrazine)
2-chloro-4,6-di(ethylamino)-1,3,5-triazine (common name simazine)
2-azido-4-*iso*propylamino-6-methylthio-1,3,5-triazine (common name aziprotryne)

K. 1-alkoxy-1-alkyl-3-phenylurea herbicides, e.g. of formula:

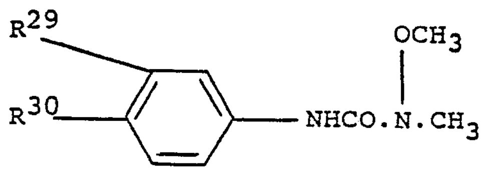

wherein $R^{29}$ is hydrogen or chloro and $R^{30}$ is chloro, bromo or ethoxy; in particular:
3-(3,4-dichlorophenyl)-1-methoxy-1-methylurea (common name linuron)
3-(4-chlorophenyl)-1-methoxy-1-methylurea (common name monolinuron)
3-(4-bromo-4-chlorophenyl)-1-methoxy-1-methylurea (common name chlorobromuron)

L. Thiolcarbamate herbicides such as S-propyl dipropylthiocarbamate (common name vernolate).

M. 1,2,4-Triazine-5-one herbicides, e.g. of formula:

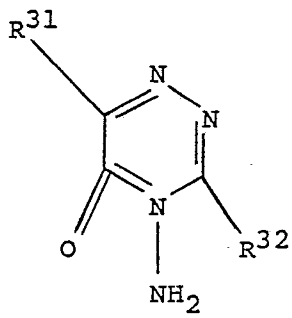

where $R^{31}$ is $C_3$—$C_6$ alkyl or cycloalkyl, or phenyl; and $R^{32}$ is methyl or methylthio; in particular: 4-amino-4,5-dihydro-3-methyl-6-phenyl-1,2,4-triazine-5-one (common name metamitron) 4-amino-6-*tert*-butyl-4,5-dihydro-3-methylthio-1,2,4- triazine-5-one (common name metribuzin).

N. Benzoic acid herbicides, e.g. those of formula:

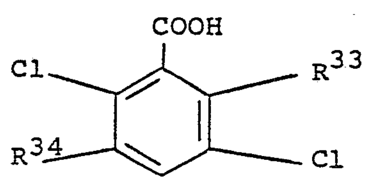

wherein $R^{33}$ is hydrogen, chloro or methoxy and $R^{34}$ is hydrogen or amino; especially 2,3,6-trichlorobenzoic

acid (common name 2,3,6-TBA); 3,6-dichloro-2-methoxybenzoic acid (common name dicamba) and 3-amino-2,5-dichlorobenzoic acid (common name chloramben).

O. Anilide herbicides, e.g. those of formula:

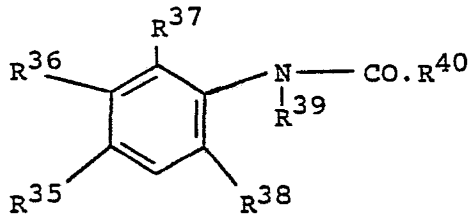

where $R^{35}$ and $R^{36}$ are hydrogen, chloro or methyl; $R^{37}$ and $R^{38}$ are H, methyl or ethyl; $R^{39}$ is hydrogen, $C_1$—$C_4$ alkyl or alkynyl, optionally substituted with $C_1$—$C_4$ alkoxy or ethoxycarbonyl; and $R^{40}$ is $C_1$—$C_6$ alkyl, cycloalkyl or alkenyl, optionally chloro substituted; particularly N-butoxymethyl-α-chloro-2′,6′-diethyl-acetanilide (common name butachlor); the corresponding N-methoxy compound (common name alachlor); the corresponding N-*iso*propyl compound (common name propachlor) and 3′,4′-dichloro-propionanilide (common name propanil).

P. Dihalobenzonitrile herbicides: for example 2,6-dichlorobenzonitrile (common name dichlorbenil); 3,5-dibromo-4-hydroxybenzonitrile (common name bromoxynil) and 3,5-diiodo-4-hydroxybenzonitrile (common name ioxynil).

Q. Haloalkanoic acid herbicides, e.g. 2,2-dichloropropionic acid (common name dalapon), trichloracetic acid (common name TCA) and salts thereof.

R. Diphenylether herbicides, e.g. 4-nitrophenyl 2′-nitro-4-trifluoromethyl phenyl ether (common name fluorodifen); methyl-5-(2,4-dichlorophenoxy)-2-nitrobenzoate (common name bifenox); 2-nitro-5-(2-chloro-4-trifluoromethylphenoxy) benzoic acid; and 2-chlorophenyl-3′-ethoxy-4′-nitro-4-trifluoromethyl phenyl ether.

S. The miscellaneous herbicides: N,N-dimethyldiphenylacetamide (common name diphenamid); N-1-naphthylphthalamic acid (common name naptalam); and 3-amino-1,2,4-triazole (common name amitrole).

T. The contact herbicides: a) a 1,1′-dimethyl-4,4-dipyridylium salt (common name paraquat), b) a 1,1′-ethylene-2,2′-dipyridylium salt (common name diquat), c) an organoarsenical herbicide e.g. monosodium methanearsonate (common name MSMA) or d) an amino acid herbicide e.g. N(phosphonmethyl)-glycine (common name glyphosate) and salts and esters thereof. In the case of mixtures with herbicides a) and b), it is preferred that the group R be an ester, e.g. a butyl ester; such mixtures have higher activity.

The preferred herbicide of formula I for use in the invention is α-[4-(5-trifluoromethyl-2-pyridyloxy)-phenoxy]propionic acid, preferably in the form of its *n*-propyl or *n*-butyl ester.

Compositions according to the invention can be used to treat a wide variety of crops. Table II below illustrates this.

TABLE II

| CROP | HERBICIDE OF FORMULA I | SUITABLE COMPLEMENTARY HERBICIDES |
|---|---|---|
| Soyabean | α-[4(5-trifluoromethyl-2-pyridyloxy)phenoxy propionic acid, *n*-butyl ester | Bentazon, chloroxuron, dinoseb, linuron, metribuzin, 2,4-DB, vernolate, alachlor, acifluorfon, oxyfluorfon |
| Cotton | " | diuron, linuron, MSMA, DSMA, fluometuron, prometryne, diphenamid, trifluralin, dinitramine, norflurazon |
| Groundnut | " | dinoseb, vernolate, bentazon, benfluralin, naptalam, fluorodiphen, diphenamid, 2,4-DB |
| Sugar beet | " | pyrazon, phenmedipham, lenacil |
| Potatoes | " | Metribuzin, linuron, monolinuron, EPTC, diphenamid |
| Cereals | α[4(3-chloro-5-trifluoro methyl-2-pyridyloxy) phenoxy propionic acid, *n*-propyl ester | 2,4-D, MCPA, MCPB, 2,4-DB, mecoprop, ioxynil, bromoxynil, dicamba, dinoterb, molinate, butachlor, propanil, triallate, chlortoluron, terbutyrne |
| Plantation Crops | α[4(3-chloro-5-trifluoro methyl-2-pyridyloxy) phenoxy propionic acid, *n*-propyl ester | simazine, atrazine, glyphosate, paraquat, diquat, bromacil, terbacil, ametryne, dalapon, dichlobenil, amitrole, monuron |

Mixtures according to the invention contain from 0.1 to 10 parts, conveniently from 0.5 to 2 parts, by weight of herbicide of formula I per part by weight of other herbicide, depending on the relative activity of the components. In a number of cases the optimum benefit from the use of the combined herbicides results when the proportion of the herbicide of formula (I) is relatively high with respect to the other herbicide. Likewise, the amount of the mixture to be applied will depend upon a number of factors, for example the particular plant species whose growth is to be inhibited, but in general an amount of from 0.1 to 5 kilograms per hectare is usually suitable. The skilled worker in the art will readily be able to determine suitable ratios and amounts for use by means of standardised routine tests, without undue experimentation.

The mixtures of the invention are preferably applied in the form of compositions, in which the active ingredients are mixed with a carrier comprising a solid or liquid diluent. Preferably the composition further comprises a surface-active agent.

The solid compositions of the invention may be for example, in the form of dusting powders, or may take the form of granules. Suitable solid diluents include, for example, kaolin, bentonite, keiselguhr, dolomite, calcium carbonate, talc, powdered magnesia and Fuller's earth.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface-active ingredients. Water or organic liquids may be used to prepare solutions, dispersions, or emulsions of the active ingredient. The liquid compositions of the invention may also contain one or more corrosion inhibitors for example lauryl isoquinolinium bromide.

Surface-active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example dodecylbenzenesulphonate, sodium, calcium and ammonium lignosulphonate, butylnaphthalene sulphonate, and a mixture of the sodium salts of diisopropyl- and triiso-propyl-naphthalenesulphonic acid. Suitable agents of the non-ionic type include, for example, the condensation products of ethylene oxide with fatty alcohols such as oleyl alcohol and cetyl alcohol, or with alkyl phenols such as octylphenol, nonylphenol and octylcresol. Other non-ionic agents are the partial esters derived from long chain fatty acids and hexitol anhydrides, for example sorbitol mono-laurate; the condensate products of the said partial esters with ethylene oxide and the lecithins.

The compositions which are to be used in the form of aqueous solutions, dispersions or emulsions are

generally supplied in the form of a concentrate containing a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment. In general concentrates may conveniently contain from 10% to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The compounds of formula I may be prepared for example by the processes disclosed in European Patent Application 78300203.3. In particular, the following route outlined in Scheme A below may be used:

Scheme A

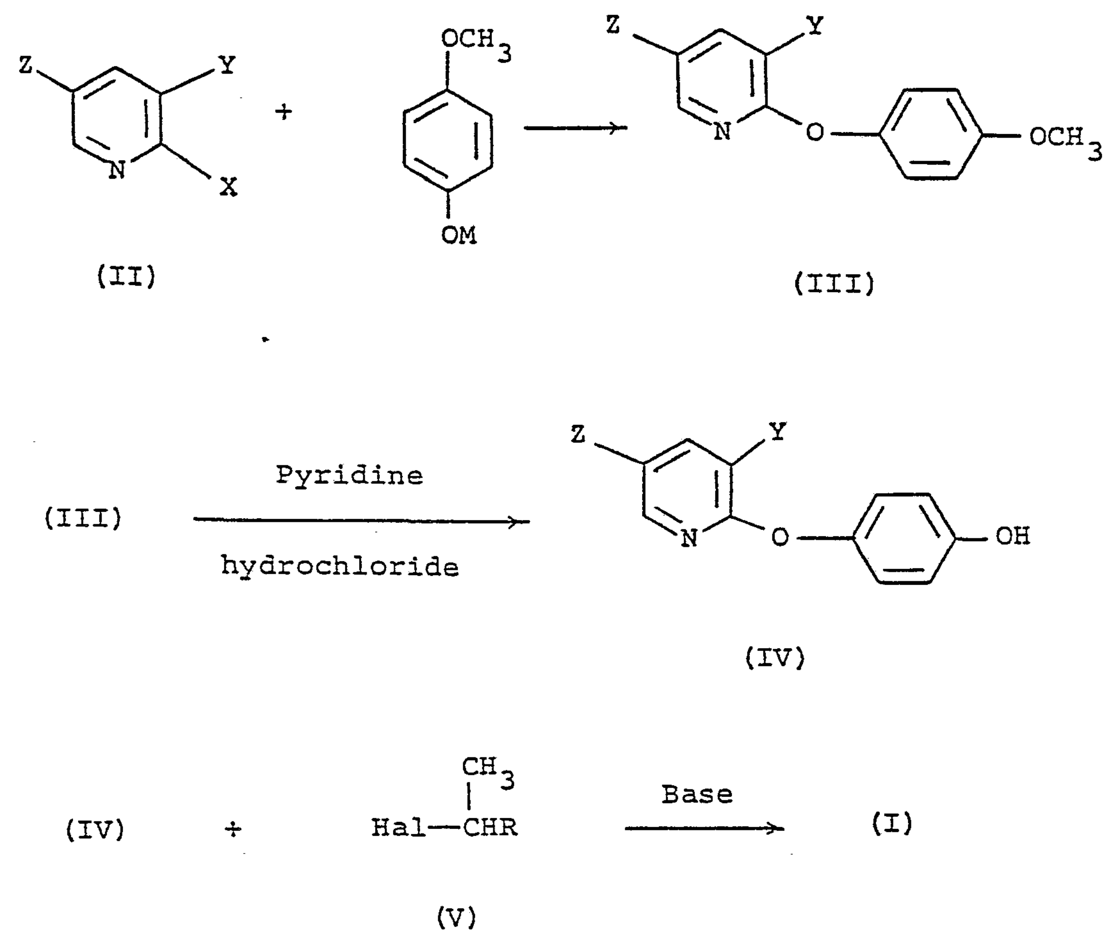


(II) (III) (IV) (V) (I)

In Scheme A, the symbols Z and Y have the meanings previously assigned to them, Hal stands for a halogen, preferably chlorine or bromine, and M is a cation, for example sodium.

In Scheme A, a suitably substituted halogeno-pyridine (II) is reacted with a metal salt of p-methoxyphenol, for example the sodium salt of p-methoxyphenol. The reaction is preferably carried out in a solvent or diluent, for example methyl ethyl ketone, tetrahydrofuran; dimethylsulphoxide or dimethylacetamide. The p-methoxyphenoxy compound (III) so obtained is then demethylated by a standard procedure, for example by heating with pyridine hydrochloride or with hydrogen bromide in acetic acid, to obtain the corresponding p-hydroxy compound (IV). This in turn is reacted in the form of its metal salt (for example the sodium or potassium salt) with the appropriate halogeno-alkanoic acid derivative (V) to obtain the required compound (I). Preferably this reaction is carried out in a solvent or diluent, for example methyl ethyl ketone.

The starting materials (II) used in the process of Scheme A may be prepared by halogenation of appropriate 2-halogeno-5- or 3-methylpyridines. Thus, 2-chloro-5-trifluoromethylpyridine may be obtained

by chlorinating 2-bromo-5-methyl-pyridine in the presence of ultra-violet to obtain 2-chloro-5-trichloromethylpyridine. The 2-chloro-5-trifluoromethylpyridine is obtainable by reacting the 2-chloro-5-trichloromethylpyridine with a fluorinating agent, for example antimony trifluoride or liquid hydrogen fluoride.

In an alternative process, 2-halogeno-3- or 5-trifluoromethyl pyridines may be obtained by reacting a 2-halogeno-3- or 5-carboxypyridine with sulphur tetrafluoride in the presence of hydrogen fluoride.

The following two experiments illustrate the preparation of compounds of formula I:

Experiment 1

This experiment illustrates the preparation of a compound of formula (I), namely, ethyl α-4(5-trifluoromethyl-2-pyridyloxy)phenoxypropionate (Compound No. 1).

(a) Preparation of 2-chloro-5-trichloromethylpyridine

2-Bromo-5-methylpyridine (27 g) in dry carbon tetrachloride (500 ml) was treated with dry hydrogen chloride to give the hydrochloride salt. The solid which separated was broken up and the mixture heated to reflux. Chlorine was passed through the boiling mixture for 8 hours with irradiation by an ultra-violet lamp. The mixture was then filtered and evaporated to a pale yellow liquid which solidified on cooling. This was identified as the required chloro compound by its nuclear magnetic resonance spectrum.

(b) Preparation of 2-chloro-5-trifluoromethylpyridine

The product from (a) (18 g) and antimony trifluoride (50 g) were heated together at 140°—145°C for 1 hour. The mixture was cooled, mixed with ice and concentrated hydrochloric acid, and extracted with ether. The extracts were washed with water, dried with magnesium sulphate, and evaporated. The products from several such preparations were combined and distilled at atmospheric pressure through a short column packed with Fenske rings. The product boiling at 124°—154°C was collected and identified as 2-chloro-5-trifluoromethylpyridine.

(c) Preparation of 2-p-methoxyphenoxy-5-trifluoromethylpyridine

The product from (b) boiling at 124°—154°C (1.82 g) was added in dry dimethylsulphoxide (15 ml) to a solution of p-methoxyphenol (1.24 g) in dimethylsulphoxide (20 ml) previously reacted with sodium hydride (0.48 g of a 50:50 oil dispersion). The mixture was stirred and heated to 60 to 65° for 5 hours, poured into ice, and extracted with ether. The ether extracts gave an oil.

(d) Preparation of 2-p-hydroxyphenoxy-5-trifluoromethylpyridine

The product from (c) (1.7 g) and excess of pyridine hydrochloride were heated together for 8 hours at 180°C. The mixture was cooled, diluted with water and 2-molar hydrochloric acid, and extracted with ether. The extracts were dried and evaporated to yield an oil identified as the required hydroxy compound.

(e) Preparation of compound no 1

The product from (d) (0.22 g), ethyl alpha-bromopropionate (0.24 g) and potassium carbonate (0.18 g) in methyl ethyl ketone (5 ml) were stirred, and heated under reflux for 2 hours. The mixture was left to cool overnight, then filtered, and the residue washed with methyl ethyl ketone. The filtrate and washings were evaporated and the remaining oil subjected to a high vacuum to remove traces of solvent. The nuclear magnetic resonance spectrum of the oil was consistent with the structure assigned and the compound was identified as compound no 1.

(f) Preparation of compound no 3

The product from (e) (0.3 g) was dissovled in *n*-pentanol (15 ml) containing concentrated sulphuric acid (2 drops). The mixture was heated to reflux for 3½ hours. The solvent was removed and the residue taken up in ether and washed with saturated sodium bicarbonate solution. The ether solution was dried and evaporated to a colourless oil which was purified by preparative scale thin layer chromatography on silica gel with an 80:20 mixture of petroleum (b.p. 60—80°C) and ether as the solvent. The nuclear magnetic resonance spectrum of the product identified it as the required pentyl ester.

(g) Preparation of compound no 2

The product from (e) (0.14 g) in isopropanol (2 ml) was stirred at room temperature for 1¾ hours with an aqueous solution of sodium hydroxide (1.6 ml of a solution containing 1 g NaOH per 100 ml water). The mixture was evaporated in a vacuum and the residue taken up in water, acidified, and extracted with ether (2 × 50 ml). The ether extracts yielded an oil identified as the required carboxylic acid.

Experiment 2

Experiment 2

This Experiment illustrates the preparation of compound No 5 of Table I.

(a) Preparation of 2-amino-3-bromo-5-methylpyridine

2-Amino-5-methylpyridine (108 g) in glacial acetic acid (300 ml) was heated to 90—100°C while bromine (160 g) in acetic acid (55 ml) was slowly added with stirring. When addition was complete, the

mixture was stirred and heated for a further 30 minutes and then allowed to cool overnight. The solid which separated was filtered off and mixed with ice and the mixture neutralised with concentrated ammonia, keeping the temperature at 0 to 5°C. The solid was collected, washed with water, and dried to give the bromo-compound.

(b) Preparation of 3-bromo-2-chloro-5-methylpyridine

The product from (a) (145 g) was dissolved in concentrated hydrochloric acid (750 ml) and water (450 ml) and the solution cooled to −10°C. Sodium nitrite (54 g) in cold water (450 ml) was added dropwise with stirring over a period of 90 minutes while the mixture was kept at −5°C. The solution was stirred for a further 2 hours, and then basified with concentrated ammonia, keeping the temperature below 20°C. The solid which separated was washed with water, dried, dissolved in ether (1500 ml) and washed with cold sodium hydroxide solution (1M; 1 litre). The ether solution was washed twice with water (1 litre portions), dried, and evaporated to give the required 3-bromo-2-chloro-5-methylpyridine.

(c) Preparation of 2,3-dichloro-5-trichloromethylpyridine

The product from (b) (64 g) in dry carbon tetrachloride (650 ml) was treated with dry hydrogen chloride. The precipitate was broken up and the suspension heated under reflux while dry chlorine was bubbled into the mixture, with illumination from an ultra-violet light source. After 4½ hours, the mixture was cooled, filtered, and the filtrate evaporated to give the required 2,3-dichloro-5-trichloromethylpyridine. The mass spectrum was consistent with the structure assigned to this compound.

(d) Preparation of 2,3-dichloro-5-trifluoromethylpyridine

The product from (c) (1.0 g) and antimony trifluoride (3.0 g) were heated together at 170—180° for 30 minutes. The mixture was then cooled, mixed with ice and water, and extracted with ether. The ether extracts gave a brown oil containing a mixture of 2,3-dichloro-5-trifluoromethylpyridine and 3-chloro-2-fluoro-5-trifluoromethylpyridine with a minor amount of 2,3-dichloro-3-chlorodifluoromethylpyridine.

(e) Preparation of 3-chloro-2-p-methoxyphenoxy-5-trifluoromethylpyridine

*p*-Methoxyphenol (1.5 g) was added to a suspension of sodium hydride (0.6 g 50% oil dispersion, washed with petroleum) in dry dimethyl sulphoxide (30 ml) and the mixture stirred for 15 minutes. A solution of the combined products (1.5 g) from several preparations carried out as described in paragraph (d), in dimethylsulphoxide (20 ml) was added to the reaction mixture and heated to 60°C for four hours. A further amount of sodium hydride (0.3 g of 50% oil dispersion, washed with petroleum), and potassium carbonate (1.38 g) was added. Heating was continued for another 4 hours. The mixture was poured into ice and water, and extracted with ether (400 ml). The ether extracts were washed with water, dilute sodium hydroxide, and water, dried, and evaporated to give the product.

(f) Preparation of 3-chloro-2-p-hydroxyphenoxy-5-trifluoromethylpyridine

The product from (e) (2 g) was heated with pyridine hydrochloride (20 g) at 170—180°C for 6 hours. The mixture was cooled, diluted with dilute hydrochloric acid, and extracted with ether. The ether extracts gave an oily solid which was purified by preparative thin layer chromatography using silica as the adsorbent and 6% ethanol-chloroform as the solvent.

(g) Preparation of compound no 5 of Table I

The product from (f) (0.16 g), ethyl alpha bromopropionate (0.3 g), and potassium carbonate (0.25 g) were heated and stirred under reflux in methyl ethyl ketone (10 ml) for 2 hours. The mixture was cooled and filtered. Evaporation of the filtrate gave an oil which was heated in a vacuum to remove traces of solvent. The oil was identified as compound no 5 by examination of its mass spectrum and its purity was confirmed by gas-liquid chromatography.

French Specification No. 2288089 discloses compositions which are mixtures of certain α-4-halopyrid-2-yloxyphenoxypropionic acids and their derivatives with other herbicides.

The following Examples illustrate compositions according to the invention.

Example 1

This Example illustrates an aqueous solution according to the invention.

| | |
|---|---|
| Compound No. 2, as sodium salt | 214 g |
| Bentazon, sodium salt | 327 g |
| Water | to 1 litre |

The other ingredients are dissolved in the water.

Example 2

This Example illustrates an aqueous solution according to the invention.

| | |
|---|---|
| Compound No. 2, as sodium salt | 160 g |
| MSMA | 300 g |
| Water | to 1 litre |

The other ingredients are dissolved in the water.

Example 3

This Example illustrates an emulsifiable composition according to the invention.

| | |
|---|---|
| Compound No. 1 | 54 g |
| Vernolate | 400 g |
| 'Lubrol' N 13 (emulsifier) | 50 g |
| 'Arylan' CA (emulsifier) | 50 g |
| 'Aromasol' H (aromatic hydrocarbon solvent) | to 1 litre |

The above ingredients are blended in a mixer.

Example 4

This Example illustrates an aqueous emulsion according to the invention.

| | |
|---|---|
| Compound No. 1 | 218 g |
| Paraquat dichloride | 138 g |
| 'Synperonic' NPE 1800 (emulsifier) | 50 g |
| 'Aromasol' H (solvent) | 100 g |
| Water | to 1 litre |

The emulsion is formed by briskly blending the ingredients.

Example 5

This Example illustrates a dispersible powder formulation according to the invention.

| | |
|---|---|
| Compound No. 7 | 100 g |
| Fluometuron | 400 g |
| 'Vanisperse' CB (dispersant) | 50 g |
| 'Aerosol' OT-B (wetter) | 20 g |
| Spestone (filler) | to 1 kg |

The above ingredients are ground together to produce a powdered mixture.

Example 6

This Example illustrates an aqueous solution according to the invention.

| | |
|---|---|
| Compound No. 2, sodium salt | 212 g |
| 2,4-DB sodium salt | 109 g |
| Water | to 1 litre |

The other ingredients are dissolved in the water.

Example 7

This Example illustrates an emulsifiable concentrate according to the invention.

| | |
|---|---|
| Compound No. 5 | 54 g |
| Phenmedipham | 100 g |
| 'Lubrol' N 13 (emulsifier) | 50 g |
| 'Arylan' CA | 50 g |
| Acetophenone | to 1 litre |

The above ingredients are blended in a mixer.

Below is given the constitution of the materials represented in the above Examples by Trade Marks.

| | |
|---|---|
| 'Lubrol' N 13 | — nonyl phenol condensed with ethylene oxide |
| 'Arylan' CA | — solution of calcium dodecyl benzene sulphonate in butanol |
| 'Aromasol' H | — aromatic hydrocarbon |
| 'Synperonic' NPE 1800 | — nonyl phenol condensed with propylene oxide and ethylene oxide |
| 'Vanisperse' CB | — sodium lignosulphonate |
| 'Aerosol'-OT-B | — sodium dioctylsulphosuccinate plus sodium benzoate |
| 'Spestone' | — china clay |

Example 8

This Example illustrates the herbicidal activity of mixtures according to the invention. Compound no. 6 of Table I was mixed with various known herbicides active against broad-leaved weeds and dispersed in water to provide spray compositions. These were then sprayed on to pot plants at the 2 to 5 leaf growth stage in the greenhouse. The rates were generally lower than those which would be applied in the field, since the young greenhouse plants are generally more tender and susceptible to herbicides than plants growing out of doors. The spray volume used was equivalent to 200 litres per hectare. Compound no. 6 was formulated as an emulsifiable concentrate of the following composition.

| Constituent | Amount (in grams per litre) |
|---|---|
| Compound no. 6 | 250 |
| Arylan CA | 2.5 |
| Geopon 365 | 2.5 |
| Aromasol H | to 1 litre |

Arylan CA comprises calcium dodecylbenzene sulphonate.

Geopon 365 comprises a condensation product of castor oil with 40 molar proportions of ethylene oxide.

Aromasol H comprises a mixture of di and trimethyl benzenes.

The known herbicides were used in the form of their commercially available formulations. A surface-active agent (Agral 90, comprising a solution of a condensation product of *p*-nonylphenol with from 7—8 molar proportions of ethylene oxide in alcohol) was added to the spray compositions so that they contained 0.1% of Agral 90 in the final spray volume.

The damage to the plants was assessed 20 days after treatment, on a scale of 0 to 9 where 0 is 0 to 10% damage and 9 is 90 to 100%. The results of the tests are given in Tables III and IV below.

TABLE III

| Added Herbicide | Application Rate of Added Herbicide | RATE OF APPLICATION OF COMPOUND NO. 6 IN KG. PER HECTARE | | | | | | | | | | | | | | | | | | | | | | | |
|---|---|---|---|---|---|---|---|---|---|---|---|---|---|---|---|---|---|---|---|---|---|---|---|---|---|
| | | 0 | | | | 0.075 | | | | 0.15 | | | | 0 | | | | 0.075 | | | | 0.15 | | | |
| | | Sb | Rp | Ca | Pi | Sb | Rp | Ca | Pi | Sb | Rp | Ca | Pi | Av | Bt | Ag | Al | Av | Bt | Ag | Al | Av | Bt | Ag | Al |
| Phenmedipham | 0 | Control | | | | 0 | 0 | 0 | 0 | 0 | 0 | 0 | 0 | Control | | | | 5 | 5 | 5 | 6 | 9 | 5 | 5 | 7 |
| | 0.5 | 0 | 4 | 4 | 4 | 0 | 1 | 5 | 5 | 0 | 0 | 3 | 5 | 0 | 0 | 0 | 0 | 5 | 4 | 5 | 6 | 7 | 4 | 5 | 6 |
| | 1.0 | 0 | 0 | 6 | 6 | 0 | <1 | 7 | 6 | 0 | 2 | 5 | 6 | 0 | 0 | 0 | 0 | 5 | 3 | 6 | 6 | 7 | 4 | 5 | 7 |
| | 2.0 | 0 | <1 | 8 | 8 | 0 | 1 | 9 | 7 | 0 | 1 | 7 | 8 | 0 | 1 | 0 | 0 | 5 | 3 | 7 | 5 | 5 | 4 | 6 | 7 |
| Metamitron | 0 | Control | | | | 0 | 0 | 0 | 0 | 0 | 0 | 0 | 0 | Control | | | | 6 | 5 | 5 | 6 | 8 | 8 | 7 | 6 |
| | 0.5 | 0 | 0 | 5 | 1 | 0 | 0 | 5 | 2 | 0 | 0 | 3 | 2 | 0 | 0 | 1 | 0 | 7 | 8 | 4 | 6 | 7 | 8 | 8 | 7 |
| | 1.0 | 0 | 6 | 8 | 5 | 0 | 2 | 9 | 4 | 0 | 2 | 8 | 4 | 2 | 0 | 0 | 1 | 6 | 7 | 5 | 6 | 7 | 8 | 7 | 7 |
| | 2.0 | 0 | 8 | 9 | 8 | 0 | 7 | 8 | 6 | 0 | 6 | 9 | 7 | 6 | 3 | 2 | 4 | 6 | 7 | 6 | 8 | 7 | 9 | 6 | 7 |
| Pyrazon | 0 | Control | | | | 0 | 0 | 0 | 0 | 0 | 0 | 1 | 0 | Control | | | | 5 | 6 | 4 | 5 | 7 | 6 | 6 | 8 |
| | 0.5 | 0 | 0 | 1 | 1 | 0 | <1 | 2 | 1 | 0 | 0 | 2 | 2 | 0 | 0 | 0 | 0 | 4 | 4 | 4 | 5 | 7 | 5 | 5 | 7 |
| | 1.0 | 0 | <1 | 3 | 4 | 0 | 1 | 3 | 4 | 0 | 1 | 3 | 3 | 1 | 0 | 0 | 0 | 5 | 4 | 5 | 5 | 7 | 5 | 4 | 6 |
| | 2.0 | 0 | 5 | 4 | 6 | 0 | 6 | 3 | 7 | 0 | 5 | 3 | 7 | 3 | 0 | 2 | 0 | 7 | 3 | 5 | 5 | 7 | 3 | 5 | 6 |

NOTE: In the tests with metamitron, compound no 6 was applied at 0.05 and 0.1 kilograms per hectare rather than 0.075 and 0.15 kilograms per hectare.

TABLE IV

| Admixed Herbicide | Application Rate of Admixed Herbicide | RATE OF APPLICATION OF COMPOUND NO. 6 IN KG. PER HECTARE | | | | | | | | | | | | | | | | | | | | | | | |
|---|---|---|---|---|---|---|---|---|---|---|---|---|---|---|---|---|---|---|---|---|---|---|---|---|---|
| | | 0 | | | | 0.075 | | | | 0.15 | | | | 0 | | | | 0.075 | | | | 0.15 | | | |
| | | Sy | Ct | Xa | Ip | Sy | Ct | Xa | Ip | Sy | Ct | Xa | Ip | Ec | Dg | Pn | Sh | Ec | Dg | Pn | Sh | Ec | Dg | Pn | Sh |
| Bentazon | 0 | Control | | | | 0 | 0 | 1 | 0 | 0 | 0 | 0 | 0 | Control | | | | 9 | 8 | 9 | 9 | 9 | 9 | 9 | 9 |
| | 0.25 | 0 | 0 | 9 | 2 | 0 | 0 | 8 | 0 | 1 | 0 | 8 | 2 | 0 | 0 | 0 | 0 | 9 | 7 | 9 | 9 | 9 | 9 | 9 | 9 |
| | 0.5 | 1 | 1 | 9 | 2 | 2 | 0 | 9 | 3 | 2 | 0 | 9 | 5 | 0 | 0 | 0 | 0 | 9 | 8 | 9 | 9 | 9 | 8 | 9 | 9 |
| | 1.0 | 4 | 0 | 9 | 9 | 4 | 0 | 9 | 8 | 4 | 1 | 9 | 5 | 0 | 0 | 0 | 0 | 9 | 8 | 9 | 9 | 9 | 8 | 9 | 9 |
| Fluometuron | 0 | Control | | | | 0 | 0 | 0 | 0 | 0 | 0 | 0 | 0 | Control | | | | 8 | 7 | 8 | 7 | 9 | 9 | 9 | 9 |
| | 0.5 | 4 | <1 | 7 | 4 | 4 | 0 | 7 | 6 | 5 | 0 | 6 | 4 | 7 | 3 | 8 | 5 | 9 | 8 | 9 | 8 | 9 | 9 | 9 | 8 |
| | 1.0 | 6 | <1 | 9 | 8 | 6 | <1 | 9 | 7 | 7 | <1 | 8 | 8 | 8 | 6 | 9 | 7 | 9 | 8 | 9 | 8 | 9 | 9 | 8 | 9 |
| | 2.0 | 8 | 0 | 9 | 8 | 8 | 0 | 9 | 7 | 9 | 0 | 9 | 9 | 9 | 8 | 9 | 8 | 9 | 9 | 9 | 9 | 9 | 9 | 9 | 9 |
| 2,4–DB | 0 | Control | | | | 0 | 0 | 0 | 1 | 0 | 0 | 0 | 0 | Control | | | | 9 | 9 | 9 | 7 | 9 | 9 | 9 | 9 |
| | 0.1 | 5 | 6 | 9 | 7 | 5 | 7 | 9 | 7 | 4 | 5 | 4 | 5 | 0 | 0 | 0 | 0 | 9 | 9 | 9 | 8 | 9 | 9 | 9 | 9 |
| | 0.2 | 5 | 7 | 9 | 9 | 5 | 8 | 9 | 7 | 5 | 8 | 9 | 7 | 1 | 0 | 1 | 0 | 9 | 9 | 9 | 7 | – | – | – | – |
| | 0.4 | 6 | 9 | 9 | 9 | 6 | 8 | 9 | 8 | 6 | 8 | 9 | 9 | 2 | 0 | 2 | 0 | 9 | 8 | 9 | 8 | – | – | – | – |

NOTES: In the tests with fluometuron, compound no 6 was tested at 0.05 and 0.1 kilograms per hectare rather than 0.075 and 0.15. Where a dash (–) appears in the Table, not test was carried out.

The names of the test plants were as follows:

| | |
|---|---|
| Sb | Sugar beet |
| Rp | Rape |
| Ca | *Chenopodium album* |
| Pi | *Polygonum aviculare* |
| Av | *Avena fatua* |
| Bt | *Bromus tectorum* |
| Ag | *Agropyron repens* |
| Al | *Alopecurus myosuroides* |
| Sy | Soya bean |
| Ct | Cotton |
| Xa | *Xanthium pensylvanium* |
| Ip | *Ipomoea purpurea* |
| Ec | *Echinochloa crus-galli* |
| Dg | *Digitaria sanguinalis* |
| Pn | *Pennisetum clandestinum* |
| Sh | *Sorghum halepense* |

## Claims

1. A herbicidal composition characterised by comprising a herbicidal compound of the formula (I)

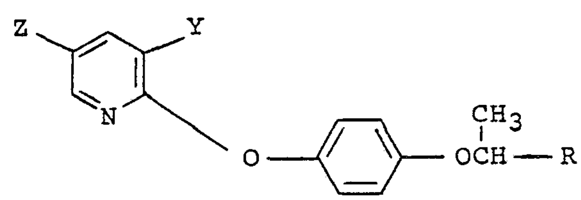

(I)

wherein at least one of Y and Z is trifluoromethyl, the other being hydrogen, fluorine, chlorine, bromine, iodine or trifluoromethyl and R is a carboxy group or a salt or ester thereof or a carboxamide or cyano group, in admixture with at least one other herbicide which is a benzo-2,1,3-thiadiazin-4-one-2,2-dioxide, a phenoxy alkanoic acid or salt, ester or amide thereof, a 3-[4-(4-halophenoxy)phenyl]-1,1-dialkyl urea, a dinitrophenol, a dinitroaniline, a phenylurea, a phenylcarbamoyloxyphenylcarbamate, a 2-phenylpyridazin-3-one, a uracil, a 1,3,5-triazine, a 1-alkoxy-1-alkyl-3-phenylurea, a thiolcarbamate, a 1,2,4-triazine-5-one, a benzoic acid, an anilide, a dihalobenzonitrile, a haloalkanoic acid, a diphenyl ether, N,N-dimethyl diphenylacetamide, N-1-naphthylphthalamic acid, 3-amino-1,2,4-triazole, a 1,1′-dimethyl-4,4-dipyridylium salt, a 1,1′-ethylene-2,2-dipyridylium salt, an organoarsenical herbicide or an aminoacid herbicide or a salt or ester thereof, the composition containing 0.1 to 10, preferably 0.5 to 2, parts by weight of the compound of formula (I) per part of said other herbicides.

2. A composition according to claim 1 characterised in that the herbicide of formula (I) is the one in which Z is trifluoromethyl, Y is hydrogen, and R is n-butoxycarbonyl or n-propoxycarbonyl.

3. A composition according to claim 1 or 2 characterised in that said other herbicide is methyl 3-m-tolylcarbamoyloxyphenylcarbamate, 4-amino-4,5-dihydro-3-methyl-6-phenyl-1,2,4-triazin-5-one, 5-amino-4-chloro-2-phenylpyridazin-3-one, 1,1-dimethyl-3- (α,α,α-trifluoro-m-tolyl)urea, 4-(2,4-dichlorophenoxy)-butyric acid or 3-isopropyl-(1H)-benzo-2,1,3-thiadiazin-4-one 2,2-dioxide.

4. A process of inhibiting the growth of unwanted plants, characterised by applying to the plants, or the

locus thereof, a composition according to any one of the preceding claims in a herbicidally effective amount.

**Patentansprüche**

1. Herbizide Zusammensetzung, dadurch gekennzeichnet, daß sie eine Verbindung der Formel (I)

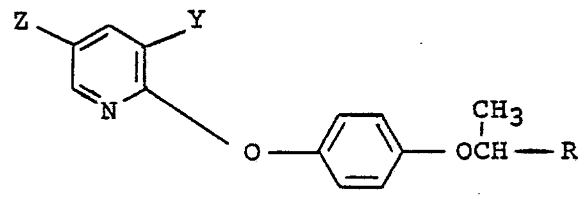

(I)

worin mindestens eines der Symbole Y und Z für Trifluoromethyl und das andere für Wasserstoff, Fluor, Chlor, Brom, Jod oder Trifluoromethyl steht and R für eine Carboxygruppe oder ein Salz oder einen Ester davon oder eine Carboxamid- oder Cyanogruppe steht, in Mischung mit mindestens einem weiteren Herbizid enthält, bei welchem es sich um ein Benzo-2,1,3-thiadiazin-4-on-2,2-dioxid, eine Phenoxyalkansäure oder ein Salz, einen Ester oder ein Amid davon, einen 3-[4-(4-Halogenophenoxy)phenyl]-1,1-dialkylharnstoff, ein Dinitrophenol, ein Dinitroanilin, einen Phenylharnstoff, ein Phenylcarbamoyloxyphenylcarbamat, ein 2-Phenylpyridazin-3-on, ein Uracil, ein 1,3,5-Triazin, einen 1-Alkoxy-1-alkyl-3-phenylharnstoff, ein Thiolcarbamat, ein 1,2,4-Triazin-5-on, eine Benzoesäure, ein Anilid, ein Dihalogenobenzonitril, eine Halogenoalkansäure, einen Diphenylether, N,N-Dimethyl-diphenylacetamid, N-1-Naphthylphthalamidsäure, 3-Amino-1,2,4-triazol, ein 1,1'-Dimethyl-4,4-dipyridyliumsalz, ein 1,1'-Ethylen-2,2-dipyridyliumsalz, ein Organoarsenherbizid oder ein Aminosäureherbizid oder ein Salz oder einen Ester davon handelt, wobei die Zusammensetzung 0,1 bis 10 Gew.-Teile, vorzugsweise 0,5 bis 2 Gew.-Teile, der Verbindung der Formel (I) je Gew.-Teil der anderen Herbizide enthält.

2. Zusammensetzung nach Anspruch 1, dadurch gekennzeichnet, daß das Herbizid der Formel (I) ein solches ist, in welchem Z für Trifluoromethyl, Y für Wasserstoff und R für n-Butoxycarbonyl oder n-Propoxycarbonyl steht.

3. Zusammensetzung nach Anspruch 1 oder 2, dadurch gekennzeichnet, daß das weitere Herbizid aus 3-m-Tolylcarbamoyloxyphenylcarbaminsäure-methylester, 4-Amino-4,5-dihydro-3-methyl-6-phenyl-1,2,4-triazin-5-on, 5-Amino-4-chloro-2-phenylpyridazin-3-on, 1,1-Dimethyl-3-(α,α,α-trifluoro-m-tolyl)harnstoff, 4-(2,4-Dichlorophenoxy)buttersäure oder 3-Isopropyl-(1H)-benzo-2,1,3-thiadiazin-4-on-2,2-dioxid besteht.

4. Verfahren zur Verhinderung des Wachstums von unerwünschten Pflanzen, dadurch gekennzeichnet, daß man auf die Pflanzen oder auf den Standort der Pflanzen eine Zusammensetzung nach einem der vorstehenden Ansprüche in einer herbizid wirksamen Menge aufbringt.

**Revendications**

1. Composition herbicide, caractérisée en ce qu'elle comprend un composé de formule (I):

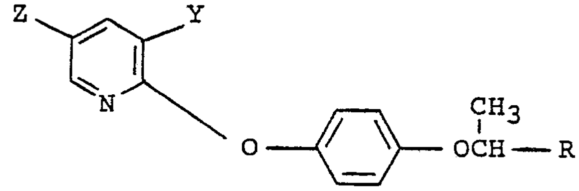

(I)

dans laquelle au moins l'un de Y et Z est un groupe trifluorométhyle, l'autre étant l'hydrogène, le fluor, le chlore, le brome, l'iode ou un groupe trifluorométhyle, et R est un groupe carboxy ou un sel de ce groupe ou un groupe carboxamide ou cyano, en mélange avec au moins un autre herbicide qui est un 2,2-dioxyde de benzo-2,1,3-thiadiazine-4-one, un acide phénoxy-alcanoïque ou un sel, ester ou amide de cet acide, une 3-[4-(4-halogénophénoxy)phényl]-1,1-dialkylurée, un dinitrophénol, une dinitroaniline, une phénylurée, un phénylcarbamoyloxyphénylcarbamate, une 2-phényl-pyridazine-3-one, un uracile, une 1,3,5-triazine, une 1-alkoxy-1-alkyl-3-phénylurée, un thiolcarbamate, une 1,2,4-triazine-5-one, un acide benzoïque, un anilide, un dihalogénobenzonitrile, un acide halogénalcanoïque, un éther de diphényle, le N,N-diméthyldiphénylacétamide, l'acide N-1-naphtylphtalamique, le 3-amino-1,2,4-triazole, un sel de 1,1'-diméthyl-4,4-dipyridylium, un sel de 1,1'-éthylène-2,2-dipyridylium, un herbicide organoarsénical ou un herbicide du type d'un aminoacide ou un sel ou ester de cet herbicide, la composition contenant 0,1 à 10, de préférence 0,5 à 2 parties en poids du composé de formule (I) par partie desdits autres herbicides.

2. Composition suivant la revendication 1, caractérisée en ce que l'herbicide de formule (I) est celui

dans lequel Z est le groupe trifluorométhyle, Y est l'hydrogène, et R est un groupe n-butoxycarbonyle ou n-propoxycarbonyle.

3. Composition suivant la revendication 1 ou 2, caractérisée en ce que l'autre herbicide est le 3-m-tolylcarbamoyloxyphénylcarbamate de méthyle, la 4-amino-4,5-dihydro-3-méthyl-6-phényl-1,2,4-triazine-5-one, la 5-amino-4-chloro-2-phénylpyridazine-3-one, la 1,1-diméthyl-3-(α,α,α-trifluoro-m-tolyl)urée, l'acide 4-(2,4-dichlorophénoxy)butyrique ou le 2,2-dioxyde de 3-isopropyl-(1H)-benzo-2,1,3-thiadiazine-4-one.

4. Procédé pour inhiber la croissance de plantes indésirables, caractérisé par l'application aux plantes ou à leur milieu d'une composition suivant l'une quelconque des revendications précédentes en une quantité efficace du point de vue herbicide.